(19) Europäisches Patentamt
European Patent Office
Office européen des brevets

(11) Veröffentlichungsnummer: **0 115 582**
**A2**

# (12) EUROPÄISCHE PATENTANMELDUNG

(21) Anmeldenummer: **83111281.8**

(22) Anmeldetag: **11.11.83**

(51) Int. Cl.³: **C 07 C 49/607**, C 07 C 45/00, C 07 C 45/67, C 11 B 9/00, A 61 K 7/46

(30) Priorität: **07.01.83 DE 3300340**

(43) Veröffentlichungstag der Anmeldung: **15.08.84**
**Patentblatt 84/33**

(84) Benannte Vertragsstaaten: **AT CH DE FR GB LI NL**

(71) Anmelder: **CHEMISCHE WERKE HÜLS AG, - RSP Patente / PB 15 - Postfach 13 20, D-4370 Marl 1 (DE)**

(72) Erfinder: **Bartmann, Martin, Dr., Burgstrasse 35, D-4350 Recklinghausen (DE)**
Erfinder: **Burzin, Klaus, Dr., Wellerfeldweg 164, D-4370 Marl (DE)**

(54) **Verwendung von Cyclododecen-2-on als Riechstoff und Verfahren zu seiner Herstellung.**

(57) Gegenstand der Erfindung sind die Verwendung von Cyclododecen-2-on (I) als Riechstoff sowie zwei neue Verfahren zur Herstellung von I. Nach dem ersten Verfahren wird Cyclododecen-2-ol einer katalytischen Dehydrierung an Kupferchromit unterzogen. Nach dem zweiten Verfahren wird das aus dem entsprechenden Bromderivat zugängliche 2-Acetoxycyclododecanon bei einer Temperatur von 400 bis 600 °C pyrolysiert.

EP 0 115 582 A2

## Verwendung von Cyclododecen-2-on als Riechstoff und Verfahren zu seiner Herstellung

Einer der wichtigsten Komponenten bekannter Riechstoffkompositionen ist das aus den Blättern der in Malaysia vorkommenden Pogestemon cablin Benth. gewonnene Patchouli-Öl. Nahezu alle bekannten Duftsprays, Deodorant-Sprays und Parfüms enthalten in Anteilen zwischen 0,1 und 50 % diese Geruchskomponente, die in entscheidender Weise imstande ist, die Note eines Duftstoffes zu prägen.

Schon seit längerer Zeit gibt es Bemühungen, das aus mindestens 24 Inhaltstoffen bestehende Konzentrat durch einen leichter herstellbaren Stoff zu ersetzen. Die bisherigen Versuche müssen als unbefriedigend angesehen werden. Zwar wird behauptet, daß 3-Methyl-9-methylen-endo-tricyclo-(5,2,1,$0^{2,6}$)-dec-3-en-8-(exo)-ol "einige der typischen Geruchsnoten reproduzieren" soll (vgl. DE-OS 31 20 700). Als Ersatz für das Patchouli-Öl kommt diese komplexe Verbindung schon deshalb nicht infrage, weil die Herstellung viel zu aufwendig ist.

Aufgabe dieser Erfindung war es, einen leicht zugänglichen Stoff zu finden, der das natürliche Patchouli-Öl ganz oder teilweise ersetzen kann.

Es wurde jetzt gefunden, daß das an sich bekannte Cyclododecen-2-on (I) diesen Anforderungen entspricht. Sein Geruch ist nicht nur dem des Patchouli-Öls sehr ähnlich und vermag letzteres zu substituieren, I ist darüber hinaus in Riechstoffkompositionen mit anderen Riechstoffen hervorragend kombinierbar.

Gegenstand der Erfindung sind gleichfalls zwei grundsätzlich verbesserte Verfahren zur Herstellung von I.

Riechstoffe cycloaliphatischer Struktur, die neben einer olefinischen Doppelbindung eine Ketogruppe enthalten, gehören zum Stand der Technik. So ist z. B. Dihydrojasmon, ein substituiertes Cyclopentanon aus der Jasminreihe, schon seit längerer Zeit bekannt (siehe Beilstein E III, Band 7, Seite 435).
Auch die Moschus-Riechstoffe, wie das natürlich vorkommende Muscon (Cyclopentadecanon), 15-Pentadecanolid und 7-Hexadecen-16-olid (vgl. Ohloff, Helv. Chim. A. 50, 705 (1967) sowie Zibeton (Cycloheptadecen-9-on) (vgl. L. Ruzicka, Helv. Chim. A. 9, 232 (1926) und das erst kürzlich isolierte Cyclopentadecen-8-on (vgl. DE-OS 29 34 678), sind Stand der Technik. Dagegen findet sich in der Literatur kein Hinweis auf die Eignung von I als Riechstoff.

In ihrem Bemühen, Exalton, Muscon und deren Homologe auf synthetischem Wege zu gewinnen, stellte eine schweizerische Forschergruppe unter anderem Cyclotridecen-2-on (M. Stoll, Helv. Chim. A. 30, 1837 (1947), Cyclopentadecen-2-on (L. Ruzicka, M. Stoll, Helv. Chim. A. 17, 1308 (1934) und Cyclohexadecen-8-on (D.R.P. 532 654) her. Die genannten ungesättigten cyclischen Ketone wurden entweder aus der entsprechenden ungesättigten Verbindung oder dem entsprechenden Hydroxyketon isoliert.

So wurde beispielsweise Cyclopentadecen mit Selendioxid in unbefriedigender Ausbeute zum entsprechenden $\alpha,\beta$-ungesättigten Keton oxidiert (L. Ruzicka, M. Stoll, Helv. Chim. A. 17, 1308 (1934). Aus dem Hydroxyketon wurden drei verschiedene Ester hergestellt und diese - ebenfalls in schlechter Ausbeute - zersetzt. Auch bei der katalytischen Dehydratation über Aluminiumoxid blieben die Ergebnisse unbefriedigend (M. Stoll, Helv. Chim. A. 30, 1837 (1947). Insbesondere treten Schwierigkeiten bei der Herstellung des Ausgangsprodukts auf.

Nach einem neueren japanischen Verfahren (JP-AS 5 148 635) kann man $\alpha,\beta$-ungesättigte cyclische Ketone mit 12 bis 18 C-Atomen dadurch erhalten, daß man die in α-Stellung durch Chlor, Brom oder Jod substituierten Ketone in Gegenwart von Hexaalkylphosphorylamid und einer alkalisch reagierenden Verbindung, wie z. B. Magnesiumhydroxid oder Lithiumcarbonat, erhitzt. Wegen der bekannten karzinogenen Eigenschaften von HMPT verbietet sich die technische Anwendung dieses Verfahrens in der Praxis.

Die Chromsäureoxidation von trans-2-Cyclododecenol (H. Nozaki et al., Tetrahydron 22, 1207 (1966) weist den Nachteil auf, daß größere Mengen von Schwermetallsalzen in verdünnter Schwefelsäure anfallen, die entweder auf kostspielige Weise aufgearbeitet oder verworfen werden müßten.

Es wurde jetzt überraschend gefunden, daß man I in guten Ausbeuten auf einem der beiden folgenden Wege erhalten kann:

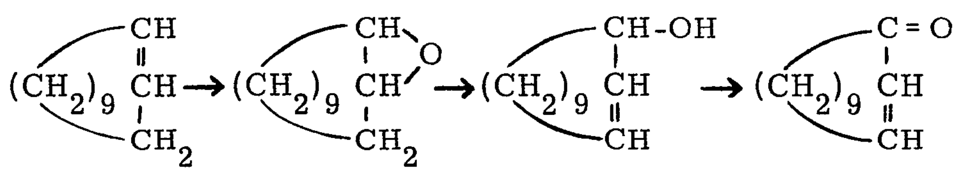

A

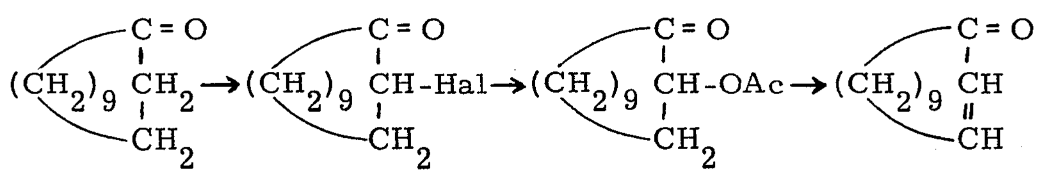

B

Es sei zunächst Weg A näher beschrieben: Die Epoxidierung entspricht Standardmethoden der organischen Chemie. Die Umlagerung kann durch n-Butyllithium oder an Lithiumphosphatkontakten bewirkt werden. Die katalytische Dehydrierung sollte bei Temperaturen zwischen 200 und 400 $^{o}C$ durchgeführt werden. Man leitet den gasförmigen Alkohol in einem Inertgasstrom über einen Kupferchromitkontakt und kondensiert das Produkt aus. Die optimalen Druckbedingungen sowie die Verweilzeit lassen sich leicht in Vergleichsversuchen bestimmen. Die Ausbeute liegt über 90 %.

Nach dem Weg B wird zunächst in α-Stellung des Ketons ein Brom- oder Jodatom eingeführt (vgl. J. Am. Chem. Soc. 80, 6039 (1958). Dieses wird im zweiten Schritt durch den Acetatrest substituiert. Die anschließende Pyrolyse, die bei einer Temperatur zwischen 400 und 600 °C durchgeführt wird, ergibt I in einer Ausbeute von über 85 %. Dieses Ergebnis ist überraschend, da thermische Zersetzungen anderer Ester des Cyclododecenon-2-ols schlechte Ergebnisse geliefert hatten (vgl. Helv. Chim. A. 30, 1837 (1947).

I fällt nach beiden Verfahren in einer Reinheit von über 98 % als cistrans-Isomerengemisch an, wobei das trans-Isomere überwiegt. Das Isomerenverhältnis ist keine kritische Größe. In dieser Form ist I technisch verwertbar. Sofern erwünscht, kann I durch Feindestillation weiter gereinigt werden. I besitzt einen an Nelkenknospen erinnernden, holzigwürzigen, warmen Geruch. Es ist hervorragend zur Substitution des natürlichen Patchouli-Öls geeignet. Von einer Verwandtschaft zu den strukturell ähnlichen Moschusriechstoffen kann daher keine Rede sein. I ist mit anderen Riechstoffen problemlos kombinierbar. Sein Anteil in Riechstoffkompositionen kann zwischen 0,5 und 50 Gewichtsprozent liegen.

Die Riechstoffkombinationen, die den erfindungsgemäßen Riechstoff enthalten, können als Parfüm oder zur Parfümierung bzw. Aromatisierung von Kosmetika, wie Cremes, Seifen, Lotionen usw., dienen. Eine weitere Verwendungsmöglichkeit ist die Geruchsverbesserung technischer Produkte, wie Wasch- und Reinigungsmittel, Desinfektionsmittel usw.

Nachfolgend werden zwei Beispiele zur Herstellung von I aufgeführt. Es folgen 2 Zusammensetzungen für Riechstoffkompositionen, in denen I enthalten ist.

Beispiel 1

Cyclododecen-2-on (Weg A)

Das durch Umlagerung von Cyclododecenepoxid gewonnene Cyclododec-2-enol wurde durch katalytische Dehydrierung an Kupferchromit[1] zum Enon oxidiert. Dazu wurde es in einer Rate von 200 g/h bei 280 °C verdampft und mit einem Stickstoffstrom von 56 l/h bei 260 °C über den Kontakt (Kontaktvolumen = 1,3 l) geleitet. Das in $CO_2$/Aceton-Kühlfallen kondensierte Produkt wurde anschließend im Ölpumpenvakuum fraktioniert.

Ausbeute: 92 %
Siedepunkt: 100 °C
IR: 1715, 1695, 1670, 1630, 990, 750 $cm^{-1}$
NMR: 6,13 - 6,97 $\delta$ (2H, -CO-CH=CH-)
2,1 - 2,60 $\delta$ (4H, $-CH_2-C=$ und $-CO-CH_2-$)
(14H, aliphatisch)

Beispiel 2a

2-Acetoxycyclododecanon (Weg B)

Eine Lösung von 175 g (0,7 Mol) 2-Bromcyclododecanon in 200 ml Eisessig wurde nach Zugabe von 63 g (0,77 Mol) Natriumacetat 6 Stunden unter Rückfluß erhitzt. Anschließend zog man den größten Teil der Essigsäure am Rotationsverdampfer ab und verdünnte mit 250 ml Wasser. Der verbleibende Rückstand wurde abgenuscht, an der Luft getrocknet und aus Ether umkristallisiert. Man erhielt 142 g 2-Acetoxycyclododecanon.

Ausbeute: 79 %
Schmelzpunkt: 84 °C
IR: 1750, 1735, 1720, 1250 $cm^{-1}$
NMR: 2,1 $\delta$ (3H, S); 5,9 $\delta$ (1H, tr)

[1] Es handelt sich dabei um einen Kupferchromkatalysator, der unter der Bezeichnung H 1044 durch die Katalysatorenwerke Hüls, Marl zu beziehen ist.

Analyse (240,34) ber.: C 69,96 H 10,07 O 19,97
gef.: C 70,1 H 10,0 O 19,9

Beispiel 2b

Cyclododecen-2-on (Weg B)

10 g 2-Acetoxycyclododecanon wurden von oben in ein mit Pyrexglasringen gefülltes, vertikal stehendes Quarzrohr von 30 cm Länge und 2 cm Durchmesser getropft. Mit Hilfe einer Rohrleitung wurde im Quarzrohr eine Temperatur von 450 °C eingestellt. Für den Stofftransport im Pyrolyserohr sorgte ein ebenfalls von oben nach unten führender Stickstoffstrom. Nach dem Verlassen des Pyrolyserohrs wurden die Reaktionsprodukte auskondensiert und mit Ether verdünnt. Danach wurde die Lösung mit $NaHCO_3$-Lösung neutral gewaschen und über $Na_2SO_4$ getrocknet. Nach dem Abziehen des Ethers am Rotationsverdampfer destillierte man den Rückstand im Ölpumpenvakuum.

Die Ausbeute betrug 6,26 g (86 %). Siedepunkt, IR- und NMR-Daten standen in Übereinstimmung mit den Werten, die nach Beispiel 1 erhalten wurden.

Beispiel 3

Blumenartige Phantasiekomposition

| | |
|---|---|
| Cyclododecen-2-on | 200 |
| 7-Hydroxy-6,7-dihydro-citronellol | 100 |
| 2-Phenylethanol | 50 |
| 3-Phenylpropanol | 50 |
| Ylang (synth.) | 50 |
| Indanmoschus | 25 |
| 2,5-Dioxa-cyclohexadecadion-(1,6) | 25 |
| Linalool | 200 |
| Linalylacetat | 100 |

| | |
|---|---|
| Benzylacetat | 50 |
| Bergamotte (synth.) | 50 |
| Gemisch aus 1-(3, 4-Epoxy-4-methyl-pentyl)-4- und 5-formylcyclohexan | 100 |
| | 1000 |

Beispiel 4

Orientalische Duftkomposition

| | |
|---|---|
| Cyclododecen-2-on | 100 |
| Cumarin | 30 |
| Bergamotteöl | 50 |
| Geraniol | 100 |
| Citronellol | 100 |
| 2-Phenyl-ethanol | 120 |
| Geraniumöl Bourbon | 30 |
| Benzoe | 20 |
| Styrax | 20 |
| Eichenmoos Extrakt | 10 |
| 2-Pentyl-zimtaldehyd | 80 |
| Benzylacetat | 100 |
| α-Methylionen | 65 |
| Sandelholzöl (ostindisch) | 20 |
| 4-tert.-Butylcyclohexylacetat | 50 |
| Lavandinöl | 50 |
| 2, 5-Dioxa-cyclohexadecadion-(1, 5) | 55 |
| | 1000 |

Substanzen wie Ylang etc., deren Struktur sich nicht unmittelbar aus ihrem Namen ergibt, sind in dem Buch von St. Arctander "Perfume and Flavor Chemicals" Montclair N. Y., USA, 1969, beschrieben.

Patentansprüche:

1. Verwendung von Cyclododecen-2-on als Riechstoff.

2. Verfahren zur Herstellung von Cyclododecen-2-on aus dem entsprechenden Enol,
dadurch gekennzeichnet,
daß man eine katalytische Dehydrierung an Kupferchromit durchführt.

3. Verfahren nach Anspruch 2,
dadurch gekennzeichnet,
daß man bei einer Temperatur zwischen 200 und 400 °C arbeitet und das gasförmige Ausgangsprodukt in einem Inertgasstrom über den Katalysator leitet.

4. Verfahren zur Herstellung von Cyclododecen-2-on aus Cyclododecanon,
dadurch gekennzeichnet,
daß man in an sich bekannter Weise das cyclische Keton in $\alpha$-Stellung halogeniert, den Halogenrest in bekannter Weise durch den Acetatrest ersetzt und das entstandene 2-Acetoxycyclododecanon pyrolysiert.

5. Verfahren nach Anspruch 4,
dadurch gekennzeichnet,
daß man als Halogen Brom verwendet und die Pyrolyse bei einer Temperatur zwischen 400 und 600 °C durchführt.

6. Riechstoffkomposition,
gekennzeichnet durch
einen Gehalt von 0,5 bis 50 % an Cyclododecen-2-on.

Ke.